(19)

Europäisches
Patentamt

European
Patent Office

Office européen
des brevets

(11) **EP 3 906 847 B1**

(12) **EUROPEAN PATENT SPECIFICATION**

(45) Date of publication and mention
of the grant of the patent:
**06.11.2024 Bulletin 2024/45**

(21) Application number: **21382412.1**

(22) Date of filing: **07.05.2021**

(51) International Patent Classification (IPC):
**A61B 5/022** *(2006.01)*   **A61B 5/026** *(2006.01)*
**A61B 5/00** *(2006.01)*   **A61B 5/0295** *(2006.01)*

(52) Cooperative Patent Classification (CPC):
**A61B 5/022; A61B 5/0261; A61B 5/0295;**
**A61B 5/7239; A61B 5/7242;** A61B 5/02233

(54) **METHOD, DEVICE AND SYSTEM FOR DETERMINING A SHIFT IN CARDIAC OUTPUT**

VERFAHREN, VORRICHTUNG UND SYSTEM ZUR BESTIMMUNG EINER VERSCHIEBUNG DES HERZZEITVOLUMENS

PROCÉDÉ, DISPOSITIF ET SYSTÈME PERMETTANT DE DÉTERMINER UN DÉCALAGE DU DÉBIT CARDIAQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.05.2020 ES 202030399**

(43) Date of publication of application:
**10.11.2021 Bulletin 2021/45**

(73) Proprietors:
• **RGB Medical Devices S.A.**
**28037 Madrid (ES)**
• **Centro Tecnologico del Mueble y La Madera
de la Region de Murcia
30510 Yecla (ES)**

(72) Inventors:
• **MAESTRE FERRIZ, Rafael**
**30510 Yecla (ES)**
• **BLEDA TOMÁS, Andrés Lorenzo**
**30510 Yecla (ES)**
• **LÓPEZ ESPINOSA, Ovidio**
**30510 Yecla (ES)**
• **CORRAL HERRANZ, Javier**
**28037 Madrid (ES)**
• **GARCÍA ALBEROLA, Arcadio**
**30510 Yecla (ES)**
• **RUIZ NOLASCO, Ricardo**
**28037 Madrid (ES)**
• **VIDAL POVEDA, Jose Arturo**
**30510 Yecla (ES)**
• **LÓPEZ GARCÍA, Pedro Carlos**
**30510 Yecla (ES)**
• **BETETA MUNÕZ, Miguel Ángel**
**30510 Yecla (ES)**

(74) Representative: **Isern Patentes y Marcas S.L.
Avda. Diagonal, 463 Bis, 2°
08036 Barcelona (ES)**

(56) References cited:
EP-A1- 1 173 093      US-A1- 2010 191 128
US-A1- 2019 200 879      US-B1- 6 402 696

EP 3 906 847 B1

**Description**

BACKGROUND OF THE INVENTION

Haemodynamic monitoring, and specifically a shift in cardiac output, is a very relevant aspect in the care and monitoring of a patient's progress, since it enables information about his or her cardiocirculatory physiopathology to be obtained. The pulmonary artery catheter (PAC) has been the most widely used technique since its introduction more than 40 years ago. Although its role in better understanding cardiovascular function is undisputed, its use has declined due to controversy surrounding its indications and limitations, as well as its obvious invasiveness. For this reason, the search for new monitoring methods has intensified. Currently, technological development provides numerous systems that explore the most important aspects of haemodynamics (preload, ventricular function, aims of haemodynamic resuscitation, etc.). These systems, like the PAC, have advantages and limitations.

Likewise, known in the current state of the art are home care units, devices for at-home care capable of measuring, detecting and calculating multiple biological parameters of a patient, and with a cost that has been significantly reduced. Home care also provides a convenient and effective method, which helps patients manage their condition on their own and thus reduces the number of hospital admissions and the consequent medical cost. For example, patent document ES2587789 discloses a portable device capable of measuring blood pressure and an electrocardiogram, operated by the patient him or herself, which can communicate the results to a server or platform with automatic data storage and analysis services to detect problems and alert the doctor. All information is accessed by the corresponding authorised persons such as doctors, the patient him or herself or family members, thus allowing continuous monitoring of the patient and his or her progress. However, this device is not able to monitor cardiac output, in other words, the haemodynamic status of the patient.

Also, the document US 2019/200879A1 discloses a no-invasive system and method for measuring blood pressure variability including a cuff pneumatically connected to a pump to inflate the cuff to be wrapped around a limb of a subject A pressure sensor is associated with the cuff for measuring cuff pressure. A photoplethysmogram sensor attached to a fingertip in the same limb of the subject and placed distal to the cuff for monitoring blood flow and recording a pulse plethysmograph signal. A control unit connected to the pressure sensor and the photoplethysmogram sensor for simultaneously recording the cuff pressure and the plethysmograph signal such that an empirical relationship is derived between the cuff pressure and an amplitude measure of the plethysmograph signal to measure short-term variation in systolic and diastolic blood pressures at a frequency corresponding to respiratory cycle.

Furthermore, patent document EP1173093 discloses a method that obtains an absolute value of cardiac output from the blood pressure signal measured invasively (in the ascending aorta, in the pulmonary artery and in the brachial, femoral and radial artery), or measured non-invasively, for example, in the arteriole of a finger, using an extremely fast pressure actuator to compensate for pressure variations in the arteriole and achieve a flat photoplethysmographic response. This method uses, on the one hand, an electromechanical mechanism that is more complex and has a faster response than the one herein proposed according to the invention, which is very sensitive to movements and tends to underestimate blood pressure, and on the other hand, a calculation method that analyses the first and second derivatives with respect to the time of the pressure signal, which requires a certain high computing capacity.

This means that there exists the opportunity to make a portable device, with improved ease of manufacture and maintenance, with a lower associated cost, as well as a method for determining a shift in cardiac output which does not require high computing capacity for the method to be easily carried out and for the device to be accessible to a greater number of users.

The present invention contributes to finding solutions and solving the existing drawback.

DESCRIPTION OF THE INVENTION

An object of the present invention is to provide a computer-implemented method by means of which a shift in cardiac output is determined from plethysmographic signals.

A plethysmographic signal, also called PS, refers to the intensity of light reflected and/or transmitted by a user's body, preferably by the arm or finger, coming from LED light emitted with a determined wavelength.

The LED light can be emitted through an LED emitting device and the light reflected and/or transmitted by a user's body can be captured through a light detector. In other words, the light detector can obtain the plethysmographic signal.

In a rest condition, in other words, when no type of pressure is being applied to the user, if an LED light is applied to a part of the user's body, the value of the plethysmographic signal is practically constant since the blood volume that circulates through the arteries and veins remains constant. Only a small periodic oscillation of the plethysmographic signal is detected due to the periodic pumping of blood through the heart.

However, if pressure is applied to the user with a cuff on a part of his or her body (such as when blood pressure is measured), and this pressure is higher than venous pressure, the venous return will close and blood will correspondingly accumulate in that part of the body. If, in addition, light is emitted towards said part of the body, the value of the plethysmographic signal will drop significantly since the volume of blood that accumulates in said part thus hinders the transmission and reflection of light.

It should be noted that the pressure value that is applied to the user when the plethysmographic signal is being measured can also be called the output pressure measurement (OPM).

Once the pressure is no longer applied, the blood volume decreases to its normal levels and, if light is applied to the same part of the body on which light was previously applied, the plethysmographic signal recovers approximately the value prior to the application of pressure.

The basis of the present invention is the comparison of plethysmographic signals obtained in spans of time during which an output pressure measurement has been applied to the user in order to determine the shift in cardiac output based on the processing of the data obtained.

Specifically, the computer-implemented method starts with a first plethysmographic signal obtained in a first span of time in which at least one first period of time is included during which an output pressure measurement has been applied to the user that is higher than venous pressure and lower than systolic pressure, and a second plethysmographic signal in a second span of time, some time after the first span of time, in which at least one second period of time is included during which an output pressure measurement has been applied to the user that is higher than venous pressure and lower than systolic pressure.

Said computer-implemented method comprises at least one step in which the shift in cardiac output is determined from at least one of the following parameters: slopes of the first and second plethysmographic signals at the loading point, magnitude of the fall of the plethysmographic signals in a first time interval and areas of the regions that lie between the line parallel to the x-axis at a height coinciding with the ordinate of the loading point and the plethysmographic signal in a second time interval; at least part of the first interval being within the first and second periods of time and part of the second interval being within the first and second periods of time within the first and second periods of time.

As mentioned, the first parameter of the plethysmographic signal from which the shift in cardiac output can be determined is the slope, Sc, of the signal at the loading point.

The loading point is understood to be the starting point of the plethysmographic signal from which the decrease of the plethysmographic signal begins in the period of time during which the output pressure measurement has been applied to the user.

Preferably, in the event that the difference in the slopes of the second plethysmographic signal and the first plethysmographic signal at the respective loading points thereof is negative, it is considered that there has been an increase in cardiac output, while if said difference is positive, it indicates that there has been a decrease in cardiac output.

The second parameter of those mentioned is the magnitude of the fall of the plethysmographic signals in a first time interval. Said fall can also be called PSf or PS fall and the time interval is preferably defined from the loading point.

In the event that the abscissa of the loading point is to and the first time interval has a duration of $t_f$, then $PSf=PS_0-PS(t_f+t_0)$; where PSo is the value of the plethysmographic signal at time $t_0$, in other words, the ordinate of the loading point.

It should be noted that a value of PS could be taken as a value of PSo at a time prior to that of the abscissa of the loading point (in other words, a time prior to applying the output pressure measurement).

Preferably, this prior time for the first plethysmographic signal is the same as the one for the second plethysmographic signal, in other words, they are at the same temporal distance from the abscissa of the loading point.

A mean value of the plethysmographic signal could also be taken as a value of PSo during the moments in times prior, preferably 5 s, to the abscissa of the loading point (in other words, a few moments in time prior to applying the output pressure measurement).

These moments in time define a time frame where, preferably, the first time interval is defined from the last moment in time of the time frame (time $t_0$).

Preferably, these prior moments in time for the first plethysmographic signal are the same as those for the second plethysmographic signal, in other words, they are at the same temporal distance from the abscissa of the loading point.

In this sense, $t_f$ can take different values, preferably in the range of 20 to 50 s and more preferably the value of 40 s. This means that the value of the first time interval is between 20 to 50 s and more preferably 40 s.

Preferably, in the event that the difference in the fall of the second plethysmographic signal and the first plethysmographic signal in a first time interval is negative, it is considered that there has been a decrease in cardiac output, while if said difference is positive, it indicates that there has been an increase in cardiac output.

The third parameter consists of the areas of the regions that lie between the line parallel to the x-axis at a height coinciding with the ordinate of the loading point and the plethysmographic signal in a second time interval.

In this sense, the duration of the second time interval can take different values, preferably in the range of 20 to 50 s and more preferably the value of 40 s. This means that the areas can delimit the regions that lie between the line parallel to the x-axis at a height coinciding with the ordinate of the loading point and the plethysmographic signal from the abscissae of the loading points thereof up to 40 seconds later.

Preferably, the second interval is defined from the abscissa of the loading point.

It should be noted that the duration of the first time interval can be the same as that of the second time interval.

Preferably, this third parameter based on the mentioned areas can be specified from the difference in the previously defined areas of each of the plethysmographic signals in said second time interval. Consequently, the difference in areas would be defined as subtraction between

- The area of the region, during the second time interval, that lies between the line parallel to the x-axis at a height coinciding with the ordinate of the loading point of the second plethysmographic signal and said second plethysmographic signal; and
- The area of the region, during the second time interval, that lies between the line parallel to the x-axis at a height coinciding with the ordinate of the loading point of the first plethysmographic signal and said second plethysmographic signal.

In the event that said difference is negative, it is considered that there has been a decrease in cardiac output. In the event that the difference is positive, it is considered that there has been an increase in cardiac output.

In a preferred embodiment of the invention, the shift in cardiac output can be determined based on the combination of the result of the shift in output obtained by two or three of these parameters (slopes at the loading point, magnitude of the fall of the plethysmographic signals in the first time interval and/or area of the regions that lie between the line parallel to the x-axis at a height coinciding with the ordinate of the loading point and the plethysmographic signal in a second time interval), each one of them being properly weighted to provide a final decision on the shift in cardiac output (it increases or decreases).

In this sense, one possible weighting would be that if the 3 parameters coincide in their assessment of the shift in cardiac output (in other words, all 3 determine that there has been an increase or decrease in cardiac output), there is maximum confidence in the result. If the parameters consisting of the magnitude of the fall of the plethysmographic signals in a first time interval and the areas of the regions that lie between the line parallel to the x-axis at a height coinciding with the ordinate of the loading point and the plethysmographic signal in a second time interval coincide in their assessment of the shift in cardiac output but differ with respect to the assessment offered by the parameter consisting of the slope at the loading point, they should be weighted so that the result offers confidence that is high but lower than the previous situation where the 3 parameters coincided in the assessment of the output. If the parameters consisting of the magnitude of the fall of the plethysmographic signals in a first time interval and the areas of the regions that lie between the line parallel to the x-axis at a height coinciding with the ordinate of the loading point and the plethysmographic signal in a second time interval do not coincide in their assessment of the shift in cardiac output (in other words, one of them determines that there has been an increase in cardiac output and another determines that there has been a decrease), the parameter of the slopes at the loading point could be used to decide on the assessment of the shift in cardiac output.

The computer-implemented method can comprise a step, preferably prior to the previous step, consisting of normalising the plethysmographic signals.

Such normalisation can be carried out by dividing the plethysmographic signal by its value at the abscissa of the loading point (in other words, at the time when the output pressure measurement is applied) or by its value at a time prior to the abscissa of the loading point (in other words, at a time prior to applying the outlet measurement pressure) or by the mean value of the plethysmographic signal during the moments in time prior, preferably 5 s, to the abscissa of the loading point (in other words, a few moments in time prior to applying the output pressure measurement).

Normalisation enables measurements taken under different circumstances to be compared with one same parameter on one same scale, offering more reliable results that are less dependent on conditions or external factors such as the pressure or area on which the device that emits the LED light and the light detector in charge of detecting the LED light are applied.

In the event that the computer-implemented method determines that there is a shift in cardiac output, it will preferably be communicated to the user either through a light or sound signal or with the corresponding interface of the computer on which it is run.

An object of the present invention is a data processing device comprising means for carrying out the computer-implemented method of the invention.

Also, the present invention would encompass a computer program comprising instructions that, when the program is run on a computer, cause the computer to carry out the steps of the computer-implemented method of the invention.

The present invention would also include a computer-readable medium comprising instructions that, when run by a computer, cause the computer to carry out the steps of the computer-implemented method of the invention.

Another object of the present invention is a method for determining a shift in cardiac output that comprises the following steps:

- Obtaining, by means of a light detector, a first plethysmographic signal in a first span of time in which at least one first period of time is included during which an output pressure measurement has been applied to the user that is higher than venous pressure and lower than systolic pressure;

- Obtaining, by means of a light detector, a second plethysmographic signal in a second span of time in which at least one second period of time is included during which an output pressure measurement has been applied to the user that is higher than venous pressure and lower than systolic pressure;
- Determining a shift in cardiac output, through processing means, from at least one of the following parameters: slopes of the first and second plethysmographic signals at the loading point, magnitude of the fall of the plethysmographic signals in a first time interval and areas of the regions that lie between the line parallel to the x-axis at a height coinciding with the ordinate of the loading point and the plethysmographic signal in a second time interval; at least part of the first interval being within the first and second periods of time and part of the second interval being within the first and second periods of time.

The determination step may also consist of determining the shift in cardiac output by means of a data processing device according to the invention, in other words, configured to carry out the computer-implemented method of the invention.

Ultimately, the shift in cardiac output is determined as set out for the computer-implemented method of the invention.

Preferably, in the event that the difference in the slopes of the second plethysmographic signal and the first plethysmographic signal at the respective loading points thereof is negative, it is considered that there has been an increase in cardiac output, while if said difference is positive, it indicates that there has been a decrease in cardiac output.

In another preferred embodiment, in the event that the difference in the fall of the second plethysmographic signal and the first plethysmographic signal in a first time interval is negative, it is considered that there has been a decrease in cardiac output, while if said difference is positive, it indicates that there has been an increase in cardiac output.

Preferably, the duration of this first time interval is in the range of 20 to 50 s and more preferably it is 40 s.

It should be noted that the fall of the plethysmographic signal can be measured as the difference between the value of the plethysmographic signal at the abscissa of the loading point and the value of the plethysmographic signal at the moment in time consisting of the sum of the abscissa of the loading point and the duration of the first time interval.

In another preferred embodiment, the fall of the plethysmographic signal can be measured as the difference between the value of the plethysmographic signal at a time prior to the abscissa of the loading point and the value of the plethysmographic signal at the end of the first time interval (moment in time resulting from adding the duration of the first time interval to the abscissa of the loading point).

Preferably, this prior time for the first plethysmographic signal is the same as the one for the second plethysmographic signal, in other words, they are at the same temporal distance from the abscissa of the respective loading points thereof.

Likewise, the fall of the plethysmographic signal can be measured as the difference between the mean value of the plethysmographic signal during moments in time prior, preferably 5 s, to the abscissa of the loading point and the value of the plethysmographic signal at the end of the first time interval.

These prior moments in time define a time frame where, preferably, the first time interval is defined from the last moment in time of the time frame.

Preferably, these prior moments in time for the first plethysmographic signal are the same as those for the second plethysmographic signal, in other words, they are at the same temporal distance from the abscissa of the respective loading points thereof.

In another preferred embodiment, following what was mentioned for the computer-implemented method, the parameter consisting of the areas of the regions that lie between the line parallel to the x-axis at a height coinciding with the ordinate of the loading point and the plethysmographic signal in a second time interval, it can be determined from the difference in said areas for each of the plethysmographic signals.

In other words, if the difference in areas between:

- the area of the region, during a second time interval, that lies between the line parallel to the x-axis at a height coinciding with the ordinate of the loading point of the second plethysmographic signal and said second plethysmographic signal; and
- the area of the region, during the second time interval, that lies between the line parallel to the x-axis at a height coinciding with the ordinate of the loading point of the first plethysmographic signal and said first plethysmographic signal

is negative, it is considered that there has been a decrease in cardiac output.

In the event that said difference in areas is positive, it indicates that there has been an increase in cardiac output.

The duration of the second time interval can take different values, preferably in the range of 20 to 50 s and more preferably the value of 40 s.

Preferably, the second time interval is defined from the abscissa of the loading point of the corresponding plethysmographic signal.

It should be noted that the duration of the first time interval can be the same as that of the second time interval.

The shift in cardiac output can be determined based on the combination of two or three of these parameters (slopes at the loading point, magnitude of the fall of the plethysmographic signals in a first time interval and/or areas of the regions that lie between the line parallel to the x-axis at a height coinciding with the ordinate of the loading point and the plethysmographic signal in a second time interval), each of them being properly weighted in the final decision.

Preferably, such weighting would be done in such a way that, if the assessment of the shift in cardiac output for each of these parameters is the same, the result should offer maximum confidence.

If, on the contrary, the assessment of the shift in cardiac output coincides with the parameters of magnitude of the fall of the plethysmographic signal in a first time interval and areas of the regions that lie between the line parallel to the x-axis at a height coinciding with the ordinate of the loading point and the plethysmographic signal in a second time interval but differs from the assessment of the shift in cardiac output according to the slopes of the plethysmographic signal at the loading point, the result should offer confidence that is acceptable but lower than the previous case.

If the assessment of the shift in cardiac output according to the parameter of the magnitude of the fall of the plethysmographic signal in a first time interval is different from the assessment of the shift in cardiac output according to the parameter of the areas of the regions that lie between the line parallel to the x-axis at a height coinciding with the ordinate of the loading point and the plethysmographic signal in a second time interval, the result will be fixed based on the assessment of the shift in cardiac output according to the parameter of the slopes at the loading point. In other words, if according to this parameter the shift in cardiac output is to increase, it will be determined as such, and if according to this parameter the shift in cardiac output is to decrease, it will be established as such.

In another preferred embodiment, the method according to the invention may comprise a step consisting of normalising the plethysmographic signals. Such normalisation can be carried out by dividing the plethysmographic signal by its value at the abscissa of the loading point, or by its value at a time prior to the abscissa of the loading point, or by the mean value of the plethysmographic signal during the moments in time prior, preferably 5s, to the abscissa of the loading point.

Another object of the present invention is a portable device capable of determining a shift in cardiac output.

The portable device of the present invention comprises processing means, an air pump, a fluidic connection port linked to the air pump, an LED emitter and a light detector, as well as a power supply to power the processing means, the LED emitter and the light detector.

Preferably, the power source is a rechargeable battery.

The processing means comprise at least one processor or processing device, but may also include storage devices, such as memory drives, necessary to be able to perform calculations required by the processing means.

The processing means are in data communication with the fluidic connection port and the light detector and can control the air pump and the power supply, as well as the LED emitter.

The processing means are configured to run the computer-implemented method of the invention mentioned above.

In a preferred embodiment, the portable device comprises a pressure sensor that is in data communication with the processing means.

By controlling the air pump, an inflatable cuff that is connected to the portable device via the fluidic connection port can be provided with an output pressure measurement higher than the venous pressure and lower than the systolic blood pressure of a user. The same portable device can also be configured to measure the user's blood pressure through the corresponding pressure sensor.

The inflatable cuff may be part of the portable device or it may be an external one of the removable type.

The LED emitter has been selected to emit light with a predetermined wavelength, preferably within the range of red light to infrared light, and the light detector is capable of detecting the spectrum of light emitted by said LED.

Preferably, the LED emitter is in data communication with the processing means such that the processing means decide on the emission of light by the LED emitter.

The light detector and the processing means are in data communication such that the processing means receive the value of light intensity detected by the light detector, in other words, the plethysmographic signal or PS.

In an operating condition of the portable device, the processing means obtain the plethysmographic signal over a span of time that includes a period of time during which a pressure of the order of venous pressure and systolic blood pressure (and preferably between the two) has been applied to the user through an inflatable cuff, said pressure being provided by the air pump and the fluidic connection port.

Preferably, the processing means is configured to determine the shift in cardiac output by running the computer-implemented method of the invention.

In a preferred embodiment, the portable device according to the invention comprises in turn a wireless connectivity module, to send the measurements of the plethysmographic signals to a server or to a smartphone, from where the received measurements can be processed and/or the computer-implemented method of the invention can be run.

In other words, the processing means of the portable device may be responsible for calculating the shift in cardiac output or transferring the necessary information to a server and/or smartphone in order to perform said calculation through the corresponding processor thereof.

In another preferred embodiment, the portable device is configured so that the haemoglobin oxygen saturation level is determined through the LED emitter and the light detector.

In this sense, the LED emitter and the light detector can be part of a pulse oximeter connected or able to connect to the portable device or integrated or able to integrate into the portable device.

Although the portable device comprises an LED emitter and a light detector, it can also include a communication port linked to the processor and configured to receive an external pulse oximeter. Preferably, said communication port would conform to the RS-232 standard or any other type of port such as USB, FireWire or Serial ATA types.

In this way, it becomes clear that both the storage and the purchase and sale of the portable device according to the invention and, where appropriate, of the inflatable cuff and/or the pulse oximeter, can be carried out separately.

Preferably, the housing of the portable biometric measuring device can have at least two integrated electrodes, linked to the processing means and configured to measure the electrocardiogram of a patient.

Likewise, the portable biometric measuring device can comprise two additional electrical connection ports, linked to the processing means, to which two wired electrodes, also configured to measure the electrocardiogram of a patient, can be connected.

Therefore, the processing means can also be configured to collect the patient's heart rate data via the electrodes, as well as measure information about his or her electrocardiogram.

Likewise, the portable device can further comprise a user interface to display the shift in cardiac output, as well as any other measurement that it is capable of taking (oxygen saturation, electrocardiogram, etc.).

As can be seen, the portable device enables the shift in cardiac output to be determined, but it can also obtain other multiple parameters of a patient, such as blood pressure, electrical activity of the heart (ECG) and haemoglobin oxygen saturation through the pulse oximeter.

BRIEF DESCRIPTION OF THE DRAWINGS

Figure 1 shows a schematic view of the inside of a basic embodiment of the portable device according to the invention.

Figure 2 shows an example of a plethysmographic signal obtained by the processor of the portable device together with the graph of the pressure applied to the cuff as a function of time.

Figure 3 shows an example of two plethysmographic signals obtained by the processor of the portable device according to the invention, normalised on the y-axis by the value of ordinates of the respective loading point thereof, and temporally aligned (abscissae) at the time of the respective loading point thereof.

Figure 4 shows a view of the outside of an embodiment of the portable device according to the invention.

Figure 5 shows a schematic view of a system for determining a shift in cardiac output according to the invention.

DESCRIPTION OF A PREFERRED EMBODIMENT

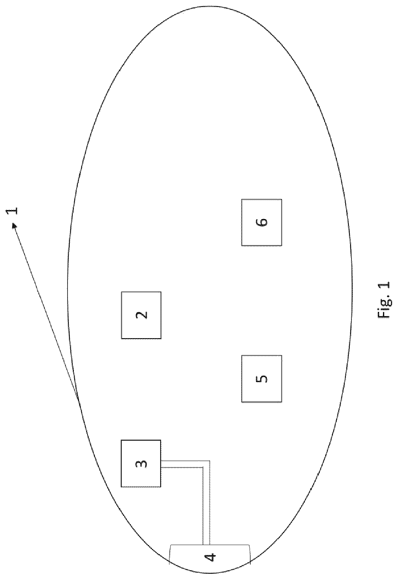

As shown in Figure 1, the portable device (1) according to the invention in the basic embodiment thereof comprises a housing that internally includes a processing device (2), an air pump (3), a fluidic connection port ( 4) linked to the air pump (3), an LED emitter (5) and a light detector (6).

Likewise, the portable device (1) further comprises a power supply (not shown in Figure 1) to power the air pump (3), the fluidic connection port (4), the LED emitter (5) and the light detector (6).

Preferably, the power source is a rechargeable battery.

The processing device (2) controls, at least, the air pump (3), the LED emitter (5) and the power supply and is in data communication with the fluidic connection port (4) and the light detector (6).

The fluidic connection port (4) is configured to receive an inflatable cuff (8) through which the user can be provided with an output pressure measurement higher than the venous pressure and lower than the systolic pressure of the user.

The processing device (2) is preferably configured to collect the corresponding measurements of pressure taken through different pressure sensors.

The LED emitter (5) is configured to emit light with a predetermined wavelength towards a part of the user's body.

The emitted light can be directed to the user's arm and/or finger. Preferably, the emitted light is infrared.

The light detector (6) is configured to receive the transmitted or reflected light emitted by the LED emitter (5) on the user's body, determining the intensity value thereof or plethysmographic signal, and to transmit the plethysmographic signal to the data processing device (2). The data processing device (2) is configured to receive plethysmographic signals.

As mentioned above, in order to determine the shift in cardiac output, it is necessary to combine the application of the output pressure measurement or OPM (higher than the venous pressure but lower than the systolic pressure of the user with a value preferably around 60 mmHg) with what is obtained from the plethysmographic signal before, during and after this pressure is applied.

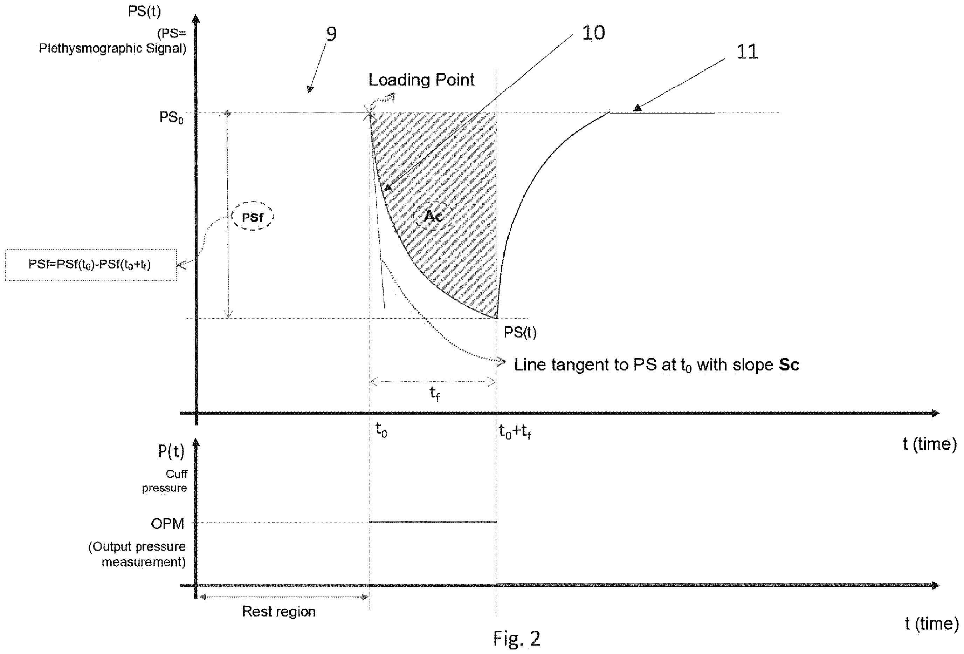

In this sense, Figure 2 shows an example of a plethysmographic signal as a function of time (PS(t)) obtained by the processing device (2) during a span of time that includes a period of time ($t_f$) during which the output pressure measurement has been applied to the user.

As can be seen, there are three well-differentiated regions.

The first area corresponds to the rest region (9) and represents the light intensity values obtained by the light detector (6) before the output pressure measurement is applied.

The second area corresponds to the transition region (10) and represents the light intensity values obtained by the light detector (6) when the output pressure measurement is being applied.

The third area corresponds to the recovery region (11) and represents the light intensity values obtained by the light detector (6) after the output pressure measurement is applied and until said values again reach those obtained before the output pressure measurement was applied.

Likewise, to determine the shift in cardiac output, it is necessary to compare a series of parameters from two plethysmographic signals ($PS_A(t)$ and $PS_B(t)$) obtained in different spans of time.

To do so, the light detector (6) is configured to obtain a first plethysmographic signal ($PS_A(t)$) in a first span of time in which at least one first period of time is included during which an output pressure measurement has been applied to the user through the inflatable cuff that is higher than venous pressure and lower than systolic pressure and a second plethysmographic signal ($PS_B(t)$) in a second span of time in which at least one second period of time is included during which an output pressure measurement has been applied to the user that is higher than the venous pressure and lower than the systolic pressure through the inflatable cuff.

In this preferred embodiment, the first period of time and the second period of time have the same temporal duration ($t_f$).

Then, the data processing device (2) determines the shift in cardiac output from at least one of the following parameters: slopes of the first and second plethysmographic signals $(\widehat{Sc_A}, \widehat{Sc_B})$ at the loading point ($t_0$, $PS_0$), magnitude of the fall of the first and second plethysmographic signals $(\widehat{PSf_A}, \widehat{PSf_B})$ in the first time interval, and areas of the regions that lie between the line parallel to the x-axis at a height coinciding with the ordinate of the loading point and the plethysmographic signal $(\widehat{Ac_A}, \widehat{Ac_B})$ in the second time interval; the first interval being within the first and second periods of time and the second interval being within the first and second periods of time.

In this preferred embodiment, the first time interval and the second time interval have the same temporal duration that coincides with the duration of the application of the output pressure measurement ($t_f$).

Preferably, the data processing device (2) is configured to determine the shift in cardiac output from at least one of the following parameters or a combination thereof:

- Difference between the value of the slope of the second plethysmographic signal at the loading point thereof and the value of the slope of the first plethysmographic signal at the loading point thereof:

  ○ If $\Delta\widehat{Sc}=\widehat{Sc_B} - \widehat{Sc_A} < 0$, it is determined that there is an increase in cardiac output,

  ○ If $\Delta\widehat{Sc}=\widehat{Sc_B} - \widehat{Sc_A} > 0$, it is determined that there is a decrease in cardiac output.

- Difference between the "PSf" value or fall of the second plethysmographic signal from the abscissa of the loading point thereof ($t_{0B}$) to $t_{0B}+t_f$ $(\widehat{PSf_B})$ and the value of "PSf' or fall of the first plethysmographic signal from the abscissa of the loading point thereof ($t_{0A}$) to $t_{0A}+t_f$ $(\widehat{PSf_A})$

  ○ If $\Delta\widehat{PSf} = \widehat{PSf_B} - \widehat{PSf_A} > 0$, an increase in output is determined,

  ○ If $\Delta\widehat{PSf} = \widehat{PSf_B} - \widehat{PSf_A} < 0$, a decrease in output is determined.

- Difference between:

  ○ the area of the region $(\widehat{Ac_B})$, during application of the output pressure measurement ($t_f$), which lies between the line parallel to the x-axis at a height coinciding with the ordinate of the loading point of the second plethysmographic signal and said second plethysmographic signal; and

  ○ the area of the region $(\widehat{Ac_A})$, during application of the output pressure measurement ($t_f$), which lies between the line parallel to the x-axis at a height coinciding with the ordinate of the loading point of the first plethysmographic signal and said first plethysmographic signal:

    ▪ If $\Delta\widehat{Ac}=\widehat{Ac_B} - \widehat{Ac_A} > 0$, an increase in output is determined,

    ▪ If $\Delta\widehat{Ac}=\widehat{Ac_B} - \widehat{Ac_A} < 0$, a decrease in output is determined.

In this preferred embodiment, the plethysmographic signals ($PS_A(t)$, $PS_B(t)$) are normalised by the value of each plethysmographic signal at the abscissa of the loading point thereof (specifically, dividing the plethysmographic signal by the corresponding PSo value thereof).

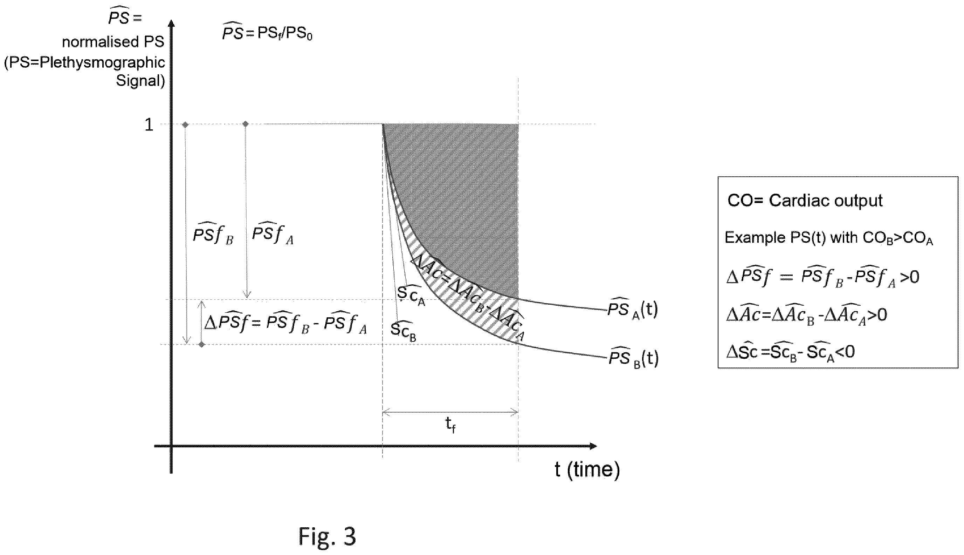

Figure 3 shows two plethysmographic signals ($PS_A(t)$, $PS_B(t)$) normalised to the value of the plethysmographic signal at the abscissa of the loading point, as well as aligned on the x-axis so that the time values of the loading points are visually overlapped, in addition to the graphic representation of each of these parameters (slope, fall and area) for each one, to make them easier to understand.

This means that the data processing device (2) can determine the shift in output only through the parameter of the slopes of the plethysmographic signals; or only through the values of the fall of the plethysmographic signals; or only through the areas of the designated regions; or through the slopes and falls of the plethysmographic signals; or through the slopes and areas of the regions; or through the falls and areas of the regions; or through the slopes, falls and areas of the regions.

Once the data processing device (2) determines the shift in cardiac output, it emits or sends some type of signal (acoustic, visual or of any other type) to the user to inform him or her of such event. In a preferred embodiment, the data processing device (2) indicates to the user the need to apply the output pressure measurement.

In another preferred embodiment, the data processing device (2) determines that there is a shift in cardiac output if the results across the three parameters (slopes, falls and areas) coincide or if at least the result of the fall and area parameters coincide.

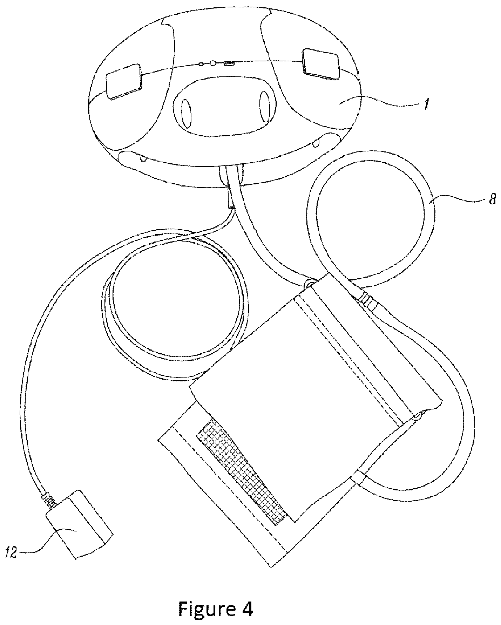

Figure 4 shows the appearance of a preferred embodiment of the portable device (1), which further includes a flexible cuff (8) configured to take the user's blood pressure.

It should be noted that in this embodiment the LED emitter (5) and the light detector (6) are integrated into a pulse oximeter (12) through which other parameters such as haemoglobin oxygen saturation can be obtained.

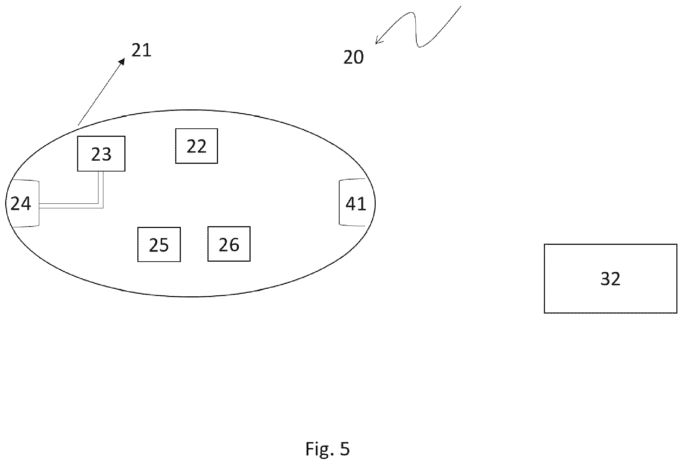

Figure 5 shows the system (20) for determining a shift in cardiac output according to the invention in the most basic variant thereof.

Specifically, the system (20) comprises a portable device (21) and a data processing device (32).

The portable device (21) comprises processing means (22), an air pump (23), a fluidic connection port (24) linked to the air pump (23), an LED emitter (25), a detector light (26) and a communication port (41).

Likewise, the portable device (21) further comprises a power supply (not shown in Figure 5) to power the air pump (23), the fluidic connection port (24), the LED emitter (25) and the light detector (26).

Preferably, the power source is a rechargeable battery.

The processing means (22) control, at least, the air pump (23), the LED emitter (25) and the power supply and is in data communication with the fluidic connection port (24) and the light detector (26).

The fluidic connection port (24) is configured to receive an inflatable cuff (28) through which the user can be provided with an output pressure measurement higher than the venous pressure and lower than the systolic pressure of the user.

The processing means (22) are preferably configured to collect the corresponding measurements of blood pressure taken through different pressure sensors.

The LED emitter (25) is configured to emit light with a predetermined wavelength towards a part of the user's body.

The emitted light can be directed to the user's arm and/or finger. Preferably, the emitted light is infrared.

The light detector (26) is configured to receive the transmitted or reflected light emitted by the LED emitter (25) on the user's body, determining the intensity value thereof or plethysmographic signal, and to transmit the plethysmographic signal to the data processing means (22). The data processing means (22) are configured to receive the plethysmographic signals.

The light detector (26) is configured to obtain a first plethysmographic signal ($PS_A(t)$) in a first period of time in which a first and second time interval is included during which an output pressure measurement has been applied to the user that is higher than venous pressure and lower than systolic pressure through the inflatable cuff, and a second plethysmographic signal ($PS_B(t)$) in a second period of time in which a first and second time interval is included during which an output pressure measurement has been applied to the user that is higher than venous pressure and lower than systolic pressure through the inflatable cuff.

In this preferred embodiment, the first period of time and the second period of time have the same temporal duration ($t_f$).

The light detector (26) is configured to transmit the plethysmographic signals to the processing means (22).

The processing means (22) are configured to receive the plethysmographic signals and to transmit them to the communication port (41).

The communication port (41) is configured to receive the plethysmographic signals and to transmit the plethysmographic signals to the data processing device (32).

The data processing device (32) is located outside the portable device (21), and can be found on a server, smartphone or any other electronic device.

The data processing device (32) is configured to receive the plethysmographic signals and to determine the shift in cardiac output from at least one of the following parameters: slopes of the first and second plethysmographic signals $(\widehat{ScA}, \widehat{ScB})$ at the loading point (to, PSo), magnitude of the fall of the first and second plethys-

mographic signals $(\widehat{PSf_A}, \widehat{PSf_B})$ in a first time interval, and areas of the regions that lie between the line parallel to the x-axis at a height coinciding with the ordinate of the loading point and the plethysmographic signal $(\widehat{Ac_A}, \widehat{Ac_B})$ in a second time interval; the first and second interval being within the first and second periods of time, respectively, during which the output pressure measurement has been applied to the user.

In this preferred embodiment, the first time interval and the second time interval have the same temporal duration that coincides with the duration of the application of the output pressure measurement ($t_f$).

Preferably, the data processing device (32) is configured to determine the shift in cardiac output from at least one of the following parameters or a combination thereof:

- Difference between the value of the slope of the second plethysmographic signal at the loading point thereof ($Sc_B$) and the value of the slope of the first plethysmographic signal at the loading point thereof ($Sc_A$):

  ○ If $\Delta\widehat{Sc}=\widehat{Sc_B} - \widehat{Sc_A}<0$, it is determined that there is an increase in cardiac output,

  ○ If $\Delta\widehat{Sc}=\widehat{Sc_B} - \widehat{Sc_A}> 0$, it is determined that there is a decrease in cardiac output.

- Difference between the "PSf" value or fall of the second plethysmographic signal from the abscissa of the loading point thereof ($t_{0B}$) to $t_{0B}+t_f$ $(\widehat{PSf_B})$ and the value of "PSf" or fall of the first plethysmographic signal from the abscissa of the loading point thereof ($t_{0A}$) to $t_{0A}+t_f$ $(\widehat{PSf_A})$

  ○ If $\Delta\widehat{PSf} = \widehat{PSf_B} - \widehat{PSf_A} > 0$, an increase in output is determined,

  ○ If $\Delta\widehat{PSf} = \widehat{PSf_B} - \widehat{PSf_A} <0$, a decrease in output is determined.

- Difference between:

  ○ the area of the region $(\widehat{Ac_B})$, during application of the output pressure measurement, which lies between the line parallel to the x-axis at a height coinciding with the ordinate of the loading point of the second plethysmographic signal and said second plethysmographic signal, and

  ○ the area of the region $(\widehat{Ac_A})$, during application of the output pressure measurement, which lies between the line parallel to the x-axis at a height coinciding with the ordinate of the loading point of the first plethysmographic signal and said first plethysmographic signal

  ▪ If $\Delta\widehat{Ac}=\widehat{Ac_B} - \widehat{Ac_A}> 0$, an increase in output is determined,

  ▪ If $\Delta\widehat{Ac}=\widehat{Ac_B} - \widehat{Ac_A}<0$, a decrease in output is determined

In this preferred embodiment, the plethysmographic signals ($PS_A(t)$, $PS_B(t)$) are normalised by the value of the plethysmographic signal at the abscissa of the loading point (specifically, by dividing the plethysmographic signal by this value).

The data processing device (32) can determine the shift in output only through the parameter of the slopes of the plethysmographic signals; or only through the values of the falls of the plethysmographic signals; or only through the areas of the regions; or through the slopes and falls of the plethysmographic signals; or through the slopes and areas of the regions; or through the falls and areas of the regions; or through the slopes, falls and areas of the regions.

Once the data processing device (32) determines the shift in cardiac output, it emits or sends some type of signal (acoustic, visual or of any other type) to the user to inform him or her of such event. In a preferred embodiment, the data processing device (32) indicates to the user the need to repeat the application of the output pressure measurement.

In another preferred embodiment, the data processing device (32) determines that there is a shift in cardiac output if the results across the three parameters (slope, fall and area) coincide or if at least the result of the fall and area parameters coincide.

The invention is defined by the appended claims.

**Claims**

1. A computer-implemented method to determine a shift in cardiac output from a first plethysmographic signal ($PS_A(t)$) in a first span of time in which at least one first period of time is included during which an output pressure measurement has been applied to the user that is higher than venous pressure and lower than systolic pressure, and a second plethysmographic signal ($PS_B(t)$) in a second span of time, some time after the first span of time, in which at least one second period of time is included during which an output pressure measurement has been applied to the user that is higher than venous pressure and lower than systolic pressure, said method comprising a step in which the shift in cardiac output is determined based on at least one of the following parameters: slopes of the first and second plethys-

mographic signals $(\widehat{Sc_A}, \widehat{Sc_B})$ at the loading point, fall of the first and second plethysmographic signals $(\widehat{PSf_A}, \widehat{PSf_B})$ in a first time interval and areas of the regions that lie between the line parallel to the x-axis at a height coinciding with the ordinate of the loading point and the plethysmographic signal $(\widehat{Ac_A}, \widehat{Ac_B})$ in a second time interval, at least part of the first interval being within the first and second periods of time and part of the second interval being within the first and second periods of time.

2. The method according to the preceding claim, wherein the shift in cardiac output is determined from at least the difference between the slope of the second plethysmographic signal $(\widehat{Sc_B})$ at the loading point thereof and the slope of the first plethysmographic signal $(\widehat{Sc_A})$ at the loading point thereof.

3. The method according to the preceding claim, wherein if the difference is negative, an increase in cardiac output is indicated.

4. The method according to any of claims 2 or 3, wherein if the difference is positive, a decrease in cardiac output is indicated.

5. The method according to any of the preceding claims, wherein the shift in cardiac output is determined from at least the difference in the fall of the second plethysmographic signal $(\widehat{PSf_B})$ and the first plethysmographic signal $(\widehat{PSf_A})$ in the first time interval.

6. The method according to claim 5, wherein the fall of the first plethysmographic signal $(\widehat{PSf_A})$ is the difference between the value of the first plethysmographic signal ($PS_A(t)$) at the end of the first time interval and the value of the first plethysmographic signal ($PS_A(t)$) at the abscissa of the loading point of the first plethysmographic signal ($PS_A(t)$), and wherein the fall of the second plethysmographic signal $(\widehat{PSf_B})$ is the difference between the value of the second plethysmographic signal ($PS_B(t)$) at the end of the first time interval and the value of the second plethysmographic signal ($PS_B(t)$) at the abscissa of the loading point of the second plethysmographic signal ($PS_B(t)$).

7. The method according to any of the preceding claims, wherein the shift in cardiac output is determined from at least the difference in the area of the region that lies between the line parallel to the x-axis at a height coinciding with the ordinate of the loading point of the second plethysmographic signal and the second plethysmographic signal in the second interval $(\widehat{Ac_B})$ and in the area of the region that lies between the line parallel to the x-axis at a height coinciding with the ordinate of the loading point of the first plethysmographic signal and the first plethysmographic signal in the second interval $(\widehat{Ac_A})$.

8. The method according to the preceding claim, wherein if the difference in areas is negative, a decrease in cardiac output is indicated.

9. The method according to any of claims 7 or 8, wherein if the difference in areas is positive, an increase in cardiac output is indicated.

10. A data processing device (2,32) comprising means for carrying out any of the methods of claims 1 to 9.

11. A computer program comprising instructions that, when the program is run on a computer, cause the computer to carry out the steps of any of the methods of claims 1 to 9.

12. A computer-readable medium comprising instructions that, when run by a computer, cause the computer to carry out any of the methods of claims 1 to 9.

13. A method for determining a shift in cardiac output that comprises the following steps:

    - Obtaining, by means of a light detector (6), a first plethysmographic signal ($PS_A(t)$) in a first span of time in which at least one first period of time is included during which an output pressure measurement has been applied to the user that is higher than venous pressure and lower than systolic pressure;
    - Obtaining, by means of a light detector (6), a second plethysmographic signal ($PS_B(t)$) in a second span of time in which at least one second period of time is included during which an output pressure measurement has been applied to the user that is higher than venous pressure and lower than systolic pressure;
    - Determining a shift in cardiac output through a data processing device (2,32) according to claim 10 and which carries out the method according to any of claims 1 to 9.

14. A portable device (1) for determining a shift in cardiac output comprising a data processing device (2,32) according to claim 10, an air pump (3), a fluidic connection port (4) linked to the air pump (3), an LED emitter (5), a light detector (6) and a power supply to power at least the processing device (2, 32), the LED emitter (5) and the light detector (6);

the fluidic connection port (4) and the light detector (6) being in data communication with the data processing device (2, 32);
the fluidic connection port (4) being configured to fluidly communicate with an inflatable cuff (8) prepared to be located on a part of a user's body;
the air pump (3) being configured to provide an output pressure measurement to the user that is higher than the venous pressure and lower than the systolic pressure of the user through the fluidic connection port (4), when said fluidic connection port (4) is in fluidic communication with the inflatable cuff (8);
the LED emitter (5) being configured to emit light with a predetermined wavelength toward a region of the user's body;
the light detector (6) being configured to obtain a first plethysmographic signal ($PS_A(t)$) of the amount of light reflected from the light emitted by the LED emitter (5) on the region of the user's body in a first span of time in which at least one first period of time is included during which an output pressure measurement has been applied to the user through the inflatable cuff (8) that is higher than venous pressure and lower the systolic pressure and a second plethysmographic signal ($PS_B(t)$) of the amount of light reflected from the light emitted by the LED emitter (5) on the region of the user's body in a second span of time, some time after the first span of time, in which at least one second period of time is included during which an output pressure measurement has been applied to the user through the inflatable cuff (8) that is higher than venous pressure and lower than systolic pressure;
the light detector (6) being in data communication with the data processing device (2, 32) and configured to transmit the plethysmographic signals ($PS_A(t)$, $PS_B(t)$) to the data processing device (2, 32);
the data processing device (2, 32) being configured to receive the plethysmographic signals ($PS_A(t)$, $PS_B(t)$).

15. A system (20) for determining a shift in cardiac output that comprises:

- a portable device (21) comprising data processing means (22), an air pump (23), a fluidic connection port (24) linked to the air pump (23), an LED emitter (25), a detector light (26) and a communication port (41) and a power supply to power at least the processing device (22), the LED emitter (25), the light detector (26) and the communication port (41);

the fluidic connection port (24) and the light detector (26) being in data communication with the data processing means (22);
the fluidic connection port (24) being configured to fluidly communicate with an inflatable cuff (28) prepared to be located on a part of the user's body;
the air pump (23) being configured to provide an output pressure measurement to the user that is higher than the venous pressure and lower than the systolic pressure of the user through the fluidic connection port (24), when said fluidic connection port (24) is in fluidic communication with the inflatable cuff (28);
the LED emitter (25) being configured to emit light with a predetermined wavelength toward a region of the user's body;
the light detector being configured to obtain a first plethysmographic signal ($PS_A(t)$) of the amount of light reflected from the light emitted by the LED emitter (25) on the region of the user's body in a first span of time in which at least one first period of time is included during which an output pressure measurement has been applied to the user through the inflatable cuff (28) that is higher than venous pressure and lower the systolic pressure and a second plethysmographic signal ($PS_B(t)$) of the amount of light reflected from the light emitted by the LED emitter (25) on the region of the user's body in a second span of time, some time after the first span of time, in which at least one second period of time is included during which an output pressure measurement has been applied to the user through the inflatable cuff (28) that is higher than venous pressure and lower than systolic pressure;
the light detector (26) being in data communication with the processing means (22) and configured to transmit the plethysmographic signals ($PS_A(t)$, $PS_B(t)$) to the processing means (22);
the processing means (22) being configured to receive the plethysmographic signals ($PS_A(t)$, $PS_B(t)$) and to transmit the plethysmographic signals ($PS_A(t)$, $PS_B(t)$) to the communication port (41);
the processing means (22) being in data communication with the communication port (41);
the communication port (41) being configured to receive the plethysmographic signals (PS_A(t), PS_B(t)) and to transmit the plethysmographic signals (PS_A(t), PS_B(t));

- A processing device (2, 32) according to claim 10, in data communication with the communication port (41) of the portable device (21) and configured to receive the plethysmographic signals (PS_A(t), PS_B(t)).

**Patentansprüche**

1. Computerimplementiertes Verfahren zur Ermittlung einer Verschiebung des Herzzeitvolumens anhand eines ersten plethysmografischen Signals (PS_A(t)) in einer ersten Zeitspanne, in der mindestens ein erster Zeitraum enthalten ist, während dem an dem Benutzer eine Ausgangsdruckmessung vorgenommen wurde, die höher ist als der Venendruck und niedriger als der systolische Druck, und eines zweiten plethysmografischen Signals (PS_B(t)) in einer zweiten Zeitspanne einige Zeit nach der ersten Zeitspanne, in der mindestens ein zweiter Zeitraum enthalten ist, während dem an dem Benutzer eine Ausgangsdruckmessung vorgenommen wurde, die höher ist als der Venendruck und niedriger als der systolische Druck, wobei das Verfahren einen Schritt umfasst, bei dem die Verschiebung des Herzzeitvolumens basierend auf mindestens einem der folgenden Parameter bestimmt wird: Steigungen des ersten und zweiten plethysmografischen Signals $(\widehat{Sc_A}, \widehat{Sc_B})$ am Lastpunkt, Abfall des ersten und zweiten plethysmografischen Signals $(\widehat{PSf_A}, \widehat{PSf_B})$ in einem ersten Zeitintervall und Flächen der Bereiche, die zwischen der Linie parallel zur x-Achse auf einer mit der Ordinate des Lastpunkts übereinstimmenden Höhe und dem plethysmografischen Signal $(\widehat{Ac_A}, \widehat{Ac_B})$ in einem zweiten Zeitintervall liegen, wobei mindestens ein Teil des ersten Intervalls innerhalb des ersten und zweiten Zeitraums liegt und ein Teil des zweiten Intervalls innerhalb des ersten und zweiten Zeitraums liegt.

2. Verfahren nach dem vorhergehenden Anspruch, wobei die Verschiebung des Herzzeitvolumens mindestens anhand der Differenz zwischen der Steigung des zweiten plethysmografischen Signals $(\widehat{Sc_B})$ an seinem Lastpunkt und der Steigung des ersten plethysmografischen Signals $(\widehat{Sc_A})$ an seinem Lastpunkt bestimmt wird.

3. Verfahren nach dem vorhergehenden Anspruch, wobei bei einer negativen Differenz eine Erhöhung des Herzzeitvolumens angezeigt wird.

4. Verfahren nach einem der Ansprüche 2 oder 3, wobei bei einer positiven Differenz eine Verringerung des Herzzeitvolumens angezeigt wird.

5. Verfahren nach einem der vorhergehenden Ansprüche, wobei die Verschiebung des Herzzeitvolumens anhand mindestens der Differenz des Abfalls des zweiten plethysmografischen Signals $(\widehat{PSf_B})$ und des ersten plethysmografischen Signals $(\widehat{PSf_A})$ im ersten Zeitintervall bestimmt wird.

6. Verfahren nach Anspruch 5, wobei der Abfall des ersten plethysmografischen Signals $(\widehat{PSf_A})$ die Differenz zwischen dem Wert des ersten plethysmografischen Signals (PS_A(t)) am Ende des ersten Zeitintervalls und dem Wert des ersten plethysmografischen Signals (PS_A(t)) an der Abszisse des Lastpunkts des ersten plethysmografischen Signals (PS_A(t)) ist und wobei der Abfall des zweiten plethysmografischen Signals $(\widehat{PSf_B})$ die Differenz zwischen dem Wert des zweiten plethysmografischen Signals (PS_B(t)) am Ende des ersten Zeitintervalls und dem Wert des zweiten plethysmografischen Signals (PS_B(t)) an der Abszisse des Lastpunkts des zweiten plethysmografischen Signals (PS_B(t)) ist.

7. Verfahren nach einem der vorhergehenden Ansprüche, wobei die Verschiebung des Herzzeitvolumens anhand mindestens der Differenz zwischen der Fläche des Bereichs, der zwischen der Linie parallel zur x-Achse auf einer mit der Ordinate des Lastpunkts des zweiten plethysmografischen Signals übereinstimmenden Höhe und dem zweiten plethysmografischen Signal im zweiten Intervall $(\widehat{Ac_B})$ liegt, und der Fläche des Bereichs, der zwischen der Linie parallel zur x-Achse auf einer mit der Ordinate des Lastpunkts des ersten plethysmografischen Signals übereinstimmenden Höhe und dem ersten plethysmografischen Signal im zweiten Intervall $(\widehat{Ac_A})$ liegt, bestimmt wird.

8. Verfahren nach dem vorhergehenden Anspruch, wobei bei einer negativen Flächendifferenz eine Verringerung des Herzzeitvolumens angezeigt wird.

9. Verfahren nach einem der Ansprüche 7 oder 8, wobei bei einer positiven Flächendifferenz eine Erhöhung des Herzzeitvolumens angezeigt wird.

10. Datenverarbeitungsgerät (2, 32), das Mittel zum Durchführen eines der Verfahren der Ansprüche 1 bis 9 umfasst.

**11.** Computerprogramm, das Anweisungen umfasst, die bei Ausführung des Programms auf einem Computer den Computer veranlassen, die Schritte eines der Verfahren der Ansprüche 1 bis 9 durchzuführen.

**12.** Computerlesbares Medium, das Anweisungen umfasst, die bei Ausführung durch einen Computer den Computer veranlassen, eines der Verfahren der Ansprüche 1 bis 9 durchzuführen.

**13.** Verfahren zum Bestimmen einer Veränderung des Herzzeitvolumens, das die folgenden Schritte umfasst:

- Erhalten eines ersten plethysmografischen Signals ($PS_A(t)$) mittels eines Lichtdetektors (6) in einer ersten Zeitspanne, in der mindestens ein erster Zeitraum enthalten ist, während dem an dem Benutzer eine Ausgangsdruckmessung vorgenommen wurde, die höher ist als der Venendruck und niedriger als der systolische Druck;

- Erhalten eines zweiten plethysmografischen Signals ($PS_B(t)$) mittels eines Lichtdetektors (6) in einer zweiten Zeitspanne, in der mindestens ein zweiter Zeitraum enthalten ist, während dem an dem Benutzer eine Ausgangsdruckmessung vorgenommen wurde, die höher ist als der Venendruck und niedriger als der systolische Druck;

- Bestimmen einer Verschiebung des Herzzeitvolumens durch ein Datenverarbeitungsgerät (2, 32) nach Anspruch 10, das das Verfahren nach einem der Ansprüche 1 bis 9 durchführt.

**14.** Tragbares Gerät (1) zum Bestimmen einer Veränderung des Herzzeitvolumens, umfassend ein Datenverarbeitungsgerät (2, 32) nach Anspruch 10, eine Luftpumpe (3), einen mit der Luftpumpe (3) verknüpften Fluidverbindungsanschluss (4), einen LED-Emitter (5), einen Lichtdetektor (6) und ein Netzteil zur Versorgung mindestens des Verarbeitungsgeräts (2, 32), des LED-Emitters (5) und des Lichtdetektors (6) mit Strom;

wobei der Fluidverbindungsanschluss (4) und der Lichtdetektor (6) in Datenkommunikation mit dem Datenverarbeitungsgerät (2, 32) stehen; wobei der Fluidverbindungsanschluss (4) so konfiguriert ist, dass er mit einer aufblasbaren Manschette (8) in Fluidverbindung steht, die dafür angefertigt ist, an einem Körperteil eines Benutzers angebracht zu werden; wobei die Luftpumpe (3) dazu konfiguriert ist, dem Benutzer über den Fluidverbindungsanschluss (4) eine Ausgangsdruckmessung bereitzustellen, die höher ist als der Venendruck und niedriger als der systolische Druck des Benutzers, wenn der Fluidverbindungsanschluss (4) in Fluidkommunikation mit der aufblasbaren Manschette (8) steht; wobei der LED-Emitter (5) dazu konfiguriert ist, Licht mit einer vorgegebenen Wellenlänge in Richtung eines Bereichs des Körpers des Benutzers zu emittieren; wobei der Lichtdetektor (6) so konfiguriert ist, dass er ein erstes plethysmografisches Signal ($PS_A(t)$) der Lichtmenge erhält, die von dem vom LED-Emitter (5) emittierten Licht auf den Bereich des Körpers des Benutzers in einer ersten Zeitspanne reflektiert wird, in der mindestens ein erster Zeitraum enthalten ist, während dem durch die aufblasbare Manschette (8) an dem Benutzer eine Ausgangsdruckmessung vorgenommen wurde, die höher ist als der Venendruck und niedriger als der systolische Druck, und ein zweites plethysmografisches Signal ($PS_B(t)$) der Lichtmenge, die von dem vom LED-Emitter (5) emittierten Licht auf den Bereich des Körpers des Benutzers in einer zweiten Zeitspanne einige Zeit nach der ersten Zeitspanne reflektiert wird, in der mindestens ein zweiter Zeitraum enthalten ist, während dem durch die aufblasbare Manschette (8) an dem Benutzer eine Ausgangsdruckmessung vorgenommen wurde, die höher ist als der Venendruck und niedriger als der systolische Druck; wobei der Lichtdetektor (6) in Datenkommunikation mit dem Datenverarbeitungsgerät (2, 32) steht und so konfiguriert ist, dass er die plethysmografischen Signale ($PS_A(t)$, $PS_B(t)$) an das Datenverarbeitungsgerät (2, 32) überträgt; wobei das Datenverarbeitungsgerät (2, 32) so konfiguriert ist, dass es die plethysmografischen Signale ($PS_A(t)$, $PS_B(t)$) empfängt.

**15.** System (20) zum Bestimmen einer Veränderung des Herzzeitvolumens, das Folgendes umfasst:

- ein tragbares Gerät (21), das Datenverarbeitungsmittel (22), eine Luftpumpe (23), einen mit der Luftpumpe (23) verknüpften Fluidverbindungsanschluss (24), einen LED-Emitter (25), einen Lichtdetektor (26) und einen Kommunikationsanschluss (41) sowie ein Netzteil zur Versorgung mindestens des Verarbeitungsgeräts (22), des LED-Emitters (25), des Lichtdetektors (26) und des Kommunikationsanschlusses (41) mit Strom umfasst;

wobei der Fluidverbindungsanschluss (24) und der Lichtdetektor (26) in Datenkommunikation mit den Datenverarbeitungsmitteln (22) stehen; wobei der Fluidverbindungsanschluss (24) so konfiguriert ist, dass er mit einer aufblasbaren Manschette (28) in Fluidverbindung steht, die dafür angefertigt ist, an einem Körperteil des Benutzers angebracht zu werden;

wobei die Luftpumpe (23) dazu konfiguriert ist, dem Benutzer über den Fluidverbindungsanschluss (24) eine Ausgangsdruckmessung bereitzustellen, die höher ist als der Venendruck und niedriger als der systolische Druck des Benutzers, wenn der Fluidverbindungsanschluss (24) in Fluidkommunikation mit der aufblasbaren Manschette (28) steht;

wobei der LED-Emitter (25) dazu konfiguriert ist, Licht mit einer vorgegebenen Wellenlänge in Richtung eines Bereichs des Körpers des Benutzers zu emittieren;

wobei der Lichtdetektor so konfiguriert ist, dass er ein erstes plethysmografisches Signal ($PS_A(t)$) der Lichtmenge erhält, die von dem vom LED-Emitter (25) emittierten Licht auf den Bereich des Körpers des Benutzers in einer ersten Zeitspanne reflektiert wird, in der mindestens ein erster Zeitraum enthalten ist, während dem durch die aufblasbare Manschette (28) an dem Benutzer eine Ausgangsdruckmessung vorgenommen wurde, die höher ist als der Venendruck und niedriger als der systolische Druck, und ein zweites plethysmografisches Signal ($PS_B(t)$) der Lichtmenge erhält, die von dem vom LED-Emitter (25) emittierten Licht auf den Bereich des Körpers des Benutzers in einer zweiten Zeitspanne einige Zeit nach der ersten Zeitspanne reflektiert wird, in der mindestens ein zweiter Zeitraum enthalten ist, während dem durch die aufblasbare Manschette (28) an dem Benutzer eine Ausgangsdruckmessung vorgenommen wurde, die höher ist als der Venendruck und niedriger als der systolische Druck;

wobei der Lichtdetektor (26) in Datenkommunikation mit den Verarbeitungsmitteln (22) steht und so konfiguriert ist, dass er die plethysmografischen Signale ($PS_A(t)$, $PS_B(t)$) an die Verarbeitungsmittel (22) überträgt;

wobei die Verarbeitungsmittel (22) so konfiguriert sind, dass sie die plethysmografischen Signale ($PS_A(t)$, $PS_B(t)$) empfangen und die plethysmografischen Signale ($PS_A(t)$, $PS_B(t)$) an den Kommunikationsanschluss (41) übertragen; wobei die Verarbeitungsmittel (22) in Datenkommunikation mit dem Kommunikationsanschluss (41) stehen;

wobei der Kommunikationsanschluss (41) so konfiguriert ist, dass er die plethysmografischen Signale ($PS_A(t)$, $PS_B(t)$) empfängt und die plethysmografischen Signale ($PS_A(t)$, $PS_B(t)$) überträgt;

- ein Verarbeitungsgerät (2, 32) nach Anspruch 10, das in Datenkommunikation mit dem Kommunikationsanschluss (41) des tragbaren Geräts (21) steht und dazu konfiguriert ist, die plethysmografischen Signale ($PS_A(t)$, $PS_B(t)$) zu empfangen.

**Revendications**

1. Procédé mis en oeuvre par ordinateur pour déterminer un changement de débit cardiaque à partir d'un premier signal pléthysmographique ($PS_A(t)$) dans un premier laps de temps où au moins une première période de temps est incluse pendant laquelle une mesure de pression de sortie a été appliquée à l'utilisateur qui est supérieure à la pression veineuse et inférieure à la pression systolique, et un second signal pléthysmographique ($PS_B(t)$) dans un second laps de temps, quelque temps après la première période de temps, où au moins une seconde période de temps est incluse pendant laquelle une mesure de pression de sortie a été appliquée à l'utilisateur qui est supérieure à la pression veineuse et inférieure à la pression systolique, ledit procédé comprenant une étape où le changement de débit cardiaque est déterminé en fonction d'au moins l'un des paramètres suivants : pentes des premier et second signaux pléthysmographiques ($\widehat{Sc_A}, \widehat{Sc_B}$) au point de charge, chute des premier et second signaux pléthysmographiques ($\widehat{PSf_A}, \widehat{PSf_B}$) dans un premier intervalle de temps et des surfaces des régions qui se situent entre la ligne parallèle à l'axe des x à une hauteur coïncidant avec l'ordonnée du point de charge et le signal pléthysmographique ($\widehat{Ac_A}, \widehat{Ac_B}$) dans un second intervalle de temps, au moins une partie du premier intervalle étant comprise dans les première et seconde périodes de temps et une partie du second intervalle étant comprise dans les première et seconde périodes de temps.

2. Procédé selon la revendication précédente, dans lequel le changement de débit cardiaque est déterminé à partir d'au moins la différence entre la pente du second signal pléthysmographique ($\widehat{Sc_B}$) au point de charge de celui-ci et la pente du premier signal pléthysmographique ($\widehat{Sc_A,}$) au point de charge de celui-ci

3. Procédé selon la revendication précédente, dans lequel la différence est négative, une augmentation du débit cardiaque est indiquée.

**4.** Procédé selon l'une quelconque des revendications 2 ou 3, dans lequel si la différence est positive, une diminution du débit cardiaque est indiquée.

**5.** Procédé selon l'une quelconque des revendications précédentes, dans lequel le changement de débit cardiaque est déterminé à partir d'au moins la différence dans la chute du second signal pléthysmographique $(\widehat{PSf_B})$ et le premier signal pléthysmographique $(\widehat{PSf_A})$ dans le premier intervalle de temps.

**6.** Procédé selon la revendication 5, dans lequel la chute du premier signal pléthysmographique $(\widehat{PSf_A})$ est la différence entre la valeur du premier signal pléthysmographique $(PS_A(t))$ à la fin du premier intervalle de temps et la valeur du premier signal pléthysmographique $(PS_A(t))$ à l'abscisse du point de charge du premier signal pléthysmographique $(PS_A(t))$, et dans lequel la chute du second signal pléthysmographique $(\widehat{PSf_B})$ est la différence entre la valeur du second signal pléthysmographique $(PS_B(t))$ à la fin du premier intervalle de temps et la valeur du second signal pléthysmographique $(PS_B(t))$ à l'abscisse du point de charge du second signal pléthysmographique $(PS_B(t))$.

**7.** Procédé selon l'une quelconque des revendications précédentes, dans lequel le changement de débit cardiaque est déterminé à partir d'au moins la différence dans la surface de la région qui se situe entre la ligne parallèle à l'axe des x à une hauteur coïncidant avec l'ordonnée du point de charge du second signal pléthysmographique et le second signal pléthysmographique dans le second intervalle $(\widehat{Ac_B})$ et dans la surface de la région qui se trouve entre la ligne parallèle à l'axe des x à une hauteur coïncidant avec l'ordonnée du point de charge du premier signal pléthysmographique et le premier signal pléthysmographique dans le second intervalle $(\widehat{Ac_A})$.

**8.** Procédé selon la revendication précédente, dans lequel si la différence de surfaces est négative, une diminution du débit cardiaque est indiquée.

**9.** Procédé selon l'une quelconque des revendications 7 ou 8, dans lequel si la différence de surfaces est positive, une augmentation du débit cardiaque est indiquée.

**10.** Dispositif de traitement de données (2, 32) comprenant des moyens pour réaliser l'un quelconque des procédés selon les revendications 1 à 9.

**11.** Programme informatique comprenant des instructions qui, lorsque le programme est exécuté sur un ordinateur, amène l'ordinateur à réaliser les étapes de l'une quelconque des procédés des revendications 1 à 9.

**12.** Support lisible par ordinateur comprenant des instructions qui, lorsqu'elles sont exécutées par un ordinateur, amènent l'ordinateur à réaliser l'un quelconque des procédés des revendications 1 à 9.

**13.** Procédé de détermination d'un changement de débit cardiaque comprenant les étapes suivantes :

- Obtention, au moyen d'un détecteur de lumière (6), d'un premier signal pléthysmographique $(PS_A(t))$ dans un premier laps de temps où au moins une première période de temps est incluse pendant laquelle une mesure de pression de sortie a été appliquée à l'utilisateur qui est supérieure à la pression veineuse et inférieure à la pression systolique ;
- Obtention, au moyen d'un détecteur de lumière (6), un second signal pléthysmographique $(PS_B(t))$ dans un second laps de temps où au moins une seconde période de temps est incluse pendant laquelle une mesure de pression de sortie a été appliquée à l'utilisateur qui est supérieure à la pression veineuse et inférieure à la pression systolique ;
- Détermination d'un changement de débit cardiaque au moyen d'un dispositif de traitement de données (2, 32) selon la revendication 10 et qui réalise le procédé selon l'une quelconque des revendications 1 à 9.

**14.** Dispositif portable (1) permettant de déterminer un changement de débit cardiaque comprenant un dispositif de traitement de données (2, 32) selon la revendication 10, une pompe à air (3), un orifice de liaison fluidique (4) relié à la pompe à air (3), un émetteur LED (5), un détecteur de lumière (6) et une alimentation électrique pour alimenter au moins le dispositif de traitement (2, 32), l'émetteur LED (5) et le détecteur de lumière (6) ;

l'orifice de liaison fluidique (4) et le détecteur de lumière (6) étant en communication de données avec le dispositif de traitement de données (2, 32) ;
l'orifice de liaison fluidique (4) étant configuré pour communiquer de manière fluidique avec un brassard gonflable (8) préparé pour être placé sur une partie du corps d'un utilisateur ;
la pompe à air (3) étant configurée pour fournir une mesure de pression de sortie à l'utilisateur qui est supérieure à la pression veineuse et inférieure à la pression systolique de l'utilisateur à travers l'orifice de liaison fluidique (4), lorsque ledit orifice de liaison fluidique (4) est en communication fluidique avec le brassard gonflable (8) ;

l'émetteur LED (5) étant configuré pour émettre de la lumière avec une longueur d'onde prédéterminée vers une région du corps de l'utilisateur ;

le détecteur de lumière (6) étant configuré pour obtenir un premier signal pléthysmographique (PS$_A$(t)) de la quantité de lumière réfléchie par la lumière émise par l'émetteur LED (5) sur la région du corps de l'utilisateur dans un premier laps de temps où au moins une première période de temps est incluse pendant laquelle une mesure de pression de sortie a été appliquée à l'utilisateur à travers le brassard gonflable (8) qui est supérieure à la pression veineuse et inférieure à la pression systolique et un second signal pléthysmographique (PS$_B$(t)) de la quantité de lumière réfléchie par la lumière émise par l'émetteur LED (5) sur la région du corps de l'utilisateur dans un second laps de temps, quelque temps après la première période de temps, où au moins une seconde période de temps est incluse pendant laquelle une mesure de pression de sortie a été appliquée à l'utilisateur à travers le brassard gonflable (8) qui est supérieure à la pression veineuse et inférieure à la pression systolique ;

le détecteur de lumière (6) étant en communication de données avec le dispositif de traitement de données (2, 32) et configuré pour transmettre les signaux pléthysmographiques (PS$_A$(t), PS$_B$(t)) au dispositif de traitement de données (2, 32) ;

le dispositif de traitement de données (2, 32) étant configuré pour recevoir les signaux pléthysmographiques (PS$_A$(t), PS$_B$(t)).

15. Système (20) permettant de déterminer un changement de débit cardiaque qui comprend :

- un dispositif portable (21) comprenant des moyens de traitement de données (22), une pompe à air (23), un orifice de liaison fluidique (24) relié à la pompe à air (23), un émetteur LED (25), un voyant de détection (26) et un orifice de communication (41) et une alimentation électrique pour alimenter au moins le dispositif de traitement (22), l'émetteur LED (25), le détecteur de lumière (26) et l'orifice de communication (41) ;

l'orifice de liaison fluidique (24) et le détecteur de lumière (26) étant en communication de données avec les moyens de traitement de données (22) ;

l'orifice de liaison fluidique (24) étant configuré pour communiquer de manière fluidique avec un brassard gonflable (28) préparé pour être placé sur une partie du corps de l'utilisateur ;

la pompe à air (23) étant configurée pour fournir une mesure de pression de sortie à l'utilisateur qui est supérieure à la pression veineuse et inférieure à la pression systolique de l'utilisateur à travers l'orifice de liaison fluidique (24), lorsque ledit orifice de liaison fluidique (24) est en communication fluidique avec le brassard gonflable (28) ;

l'émetteur LED (25) étant configuré pour émettre de la lumière avec une longueur d'onde prédéterminée vers une région du corps de l'utilisateur ;

le détecteur de lumière étant configuré pour obtenir un premier signal pléthysmographique (PS$_A$(t)) de la quantité de lumière réfléchie par la lumière émise par l'émetteur LED (25) sur la région du corps de l'utilisateur dans un premier laps de temps où au moins une première période de temps est incluse pendant laquelle une mesure de pression de sortie a été appliquée à l'utilisateur à travers le brassard gonflable (28) qui est supérieure à la pression veineuse et inférieure à la pression systolique et un second signal pléthysmographique (PS$_B$(t)) de la quantité de lumière réfléchie par la lumière émise par l'émetteur LED (25) sur la région du corps de l'utilisateur dans un second laps de temps,

quelque temps après la première période de temps, où au moins une seconde période de temps est incluse pendant laquelle une mesure de pression de sortie a été appliquée à l'utilisateur à travers le brassard gonflable (28) qui est supérieure à la pression veineuse et inférieure à la pression systolique ;

le détecteur de lumière (26) étant en communication de données avec les moyens de traitement (22) et configuré pour transmettre les signaux pléthysmographiques (PS$_A$(t), PS$_B$(t)) aux moyens de traitement (22) ;

les moyens de traitement (22) étant configurés pour recevoir les signaux pléthysmographiques (PS$_A$(t), PS$_B$(t)) et pour transmettre les signaux pléthysmographiques (PS$_A$(t), PS$_\beta$(t)) à l'orifice de communication (41) ;

les moyens de traitement (22) étant en communication de données avec l'orifice de communication (41) ;

l'orifice de communication (41) étant configuré pour recevoir les signaux pléthysmographiques ($PS_A(t)$, $PS_B(t)$) et pour transmettre les signaux pléthysmographiques ($PS_A(t)$, $PS_B(t)$) ;

- Dispositif de traitement (2, 32) selon la revendication 10, en communication de données avec l'orifice de communication (41) du dispositif portable (21) et configuré pour recevoir les signaux pléthysmographiques ($PS_A(t)$, $PS_B(t)$).

Fig. 1

Fig. 2

Fig. 3

Figure 4

Fig. 5

**EP 3 906 847 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- ES 2587789
- US 2019200879 A1
- EP 1173093 A